# EUROPÄISCHE PATENTSCHRIFT

(11) **EP 4 175 082 B1**
(45) Veröffentlichungstag und Bekanntmachung des Hinweises auf die Patenterteilung: **19.06.2024**
(21) Anmeldenummer: 22203511.5
(22) Anmeldetag: 25.10.2022
(51) Int. Cl.: H01T 23/00, A61L 9/22, H01T 19/00

(54) **VORRICHTUNG ZUR IONISERUNG VON UMGEBUNGSLUFT**
DEVICE FOR IONIZING AMBIENT AIR
DISPOSITIF D'IONISATION DE L'AIR AMBIANT

(30) Priorität: 26.10.2021 DE 102021127875
(43) Veröffentlichungstag der Anmeldung: 03.05.2023
(73) Patentinhaber: Krömker Holding GmbH, 31675 Bückeburg (DE); Timon Schorling Holding GmbH, 31737 Rinteln (DE)
(72) Erfinder: Krömker, Wilfried, 31675 Bückeburg (DE); Schorling, Timon, 20257 Hamburg (DE)
(74) Vertreter: Kröncke, Rolf

(56) Entgegenhaltungen:
- EP-A1- 0 231 436
- WO-A1-97/09268
- DE-A1-102005 056 726
- US-A1- 2003 137 794
- US-A1- 2004 256 225
- US-A1- 2008 030 918
- US-A1- 2017 333 587

## Beschreibung

Die Erfindung betrifft eine Vorrichtung zur Ionisierung von Umgebungsluft mit einem Behälter aus elektrisch isolierendem Material, einer im Innenraum des Behälters angeordneten Innenelektrode und einer an der Außenseite des Behälters angeordneten Außenelektrode, und mit einer Ansteuerelektronik, die mit der Innenelektrode und der Außenelektrode zu Beaufschlagung mit einer Versorgungsspannung verbunden ist.

Die Vorrichtung ist insbesondere eingerichtet und geeignet, Hydroxylradikale -OH und Ozon Os zu erzeugen.

EP 3 120 185 B1 offenbart eine Ionisierungsvorrichtung, die einen Glaskolben und eine innerhalb des Glaskolbens angeordnete innere Elektrode und eine auf der äu-ßeren Seite des Glaskolbens angeordnete äußere Elektrode hat. Der Glaskolben ist mit einem Polymerfilm bedeckt. Damit wird sichergestellt, dass Glassplitter festgehalten werden und hierdurch keine Gefährdung auftritt.

GB 2 093 638 A offenbart einen lonengenerator mit einer konzentrischen Innenelektrode und einer diese zylinderförmig umgebenden Außenelektrode, wobei ein aktiviertes Aktivkohlegewebe in dem Zwischenraum zwischen der Innenelektrode und der Außenelektrode angeordnet ist.

Ebenso offenbart EP 0 789 666 B1 eine Vorrichtung zur Ozonerzeugung, bei der der Spalt zwischen zwei Elektroden durch eine elektrisch leitfähige und wärmeleitfähige, gasdurchlässige Anordnung ausgefüllt ist, die aus Draht als Geflecht, Gewebe oder Gestrick ausgebildet ist.

US 2008/0030918 A1 beschreibt einen Ionisator mit Entladungsnadeln und einem Drahtgitter, an die eine Spannung angelegt wird.

US 2003/0137794 A1 offenbart einen Ionengenerator mit einer Glasröhre. Für den Aufbau der Elektroden wird eine Masseelektrode aus einem Drahtgeflecht aus Edelstahl und eine Anwendungselektrode aus einer Platte aus Edelstahl vorgeschlagen.

EP 0 231 436 A1 beschreibt eine Sprühelektrode, die Aluminiumgranulat als Füllung eines am sprühenden Ende geschlossenen Rohrs aus Quarz oder einem vergleichbaren Isoliermaterial aufweist.

US 2004/0256225 A1 beschreibt eine Vorrichtung zur dielektrisch behinderten Entladung mit einer Glasröhre, das mit grobkörnigem Carbon-Granulat mit einer Korngröße von 3-5 mm x 1 mm gefüllt wird.

WO 97/09268 A1 offenbart eine Vorrichtung zur Erzeugung von Ozon mit einer stabförmigen Innenelektrode, einer zylinderförmigen Außenelektrode und einem zwischenliegenden zylinderförmigen Dielektrikum. Der Spalt zwischen der Innenelektrode und dem Dielektrikum und der Spalt zwischen der Außenelektrode und dem Dielektrikum ist durch eine elektrisch leitfähige, wärmeleitfähige und gasdurchlässige Anordnung ausgefüllt. Ein Gasstrom wird durch die Spalte der rohrförmigen Vorrichtung hindurchgeführt und es wird Ozon durch dielektrisch behinderte Entladung an dem Dielektrikum erzeugt. Die gasdurchlässige Anordnung in den Spalten kann Späne, Granulat, Draht, Geflecht, Gewebe, Vlies, Gestrick oder poröse Granulate sein, wobei eine Gasdurchlässigkeit sichergestellt werden muss, so dass das Spaltvolumen nicht mehr als 50% ausgefüllt wird.

US 2017/333587 A1 offenbart einen Ozongenerator, der aus einer Glasröhre, einer inneren Elektrode aus gerolltem perforiertem Aluminiumblatt und einer äußeren Elektrode aus einem rohrförmigen Edelstahl-Drahtgeflecht gebildet ist.

DE 10 2005 056 726 A1 zeigt eine Vorrichtung zur Ozonherstellung mittels dielektrisch behinderter Entladung mit einem Trägerkörper aus Natronkalkglas, in den eine Edelstahlwolle als Innenelektrode eingebracht ist, die für einen geringen Energieverbrauch maßgeblich sein soll.

Ausgehend hiervon ist es Aufgabe der vorliegenden Erfindung, eine verbesserte Vorrichtung zur Ionisierung von Umgebungsluft zu schaffen, die zu einer höheren Ausbeute von Radikalen, insbesondere von Hydroxylradikalen, und wirksamem Ozon unter Reduzierung schädlicher Stickstoffverbindungen NOx führt.

Die Aufgabe wird mit der Vorrichtung mit dem Merkmal des Anspruchs1 gelöst. Vorteilhafte Ausführungsformen sind in den Unteransprüchen beschrieben.

Es wird vorgeschlagen, dass ein elektrisch leitfähiges Granulat mit einer mittleren Korngröße im Bereich von 10 bis 450 µm in dem Innenraum des Behälters angeordnet ist, wobei das Granulat bei eingesteckter Innenelektrode zusammengepresst und die Innenelektrode von dem Granulat umgeben ist.

Im Gegensatz zu dem Ausfüllen des Behälters mit elektrisch leitfähigem Vollmaterial, einem Drahtgeflecht oder lediglich Luft hat sich überraschend gezeigt, dass feinkörniges zusammengepresstes Granulat zu einem verbesserten Wirkungsgrad führt.

Die zur Ausbildung von Coronaentladungen wirksamen Luftstrecken bei einem Drahtgitter sind dort nicht vorhanden. Sie sind aber auch nicht vollständig, wie bei Vollmaterial, entfernt. Die bei einem feinkörnigen Granulat, wie insbesondere einem feinkörnigen Pulver, immer vorhandenen sehr kurzen, extrem kleinen Zwischenräume haben im Vergleich zu Vollmaterial den Effekt eines höheren Ionisierungswirkungsgrades. Auch im Vergleich zu Fasermaterial und Gewebe als Zwischenmaterial mit größeren Lufteinschlüssen führt eine Granulatfüllung mit elektrisch leitfähigen Partikeln ebenfalls zu einem höheren Ionisierungsgrad.

Das Granulat kann beispielsweise aluminiumhaltige, kupferhaltige, edelstahlhaltige, titanhaltige, graphithaltige und/oder magnesiumhaltige Partikel enthalten. Diese können auch in Form von Legierungen vorhanden sein, wie beispielsweise Bronze, Messing und dergleichen.

Erfindungsgemäß wird ein Granulat mit einer mittleren Korngröße im Bereich von 10 bis 450 µm genutzt.

So haben sich Kupferpulver mit einer Korngrößenverteilung im Bereich von 100 bis 450 µm als geeignet herausgestellt. Ebenso kann Aluminiumpulver oder Edelstahlpulver mit einer Korngröße im Bereich von 7 bis 450 µm und bevorzugt in einem Bereich von 10 bis 150 µm verwendet werden.

Das Granulat wird nach Einstecken der Innenelektrode in das Granulat zusammengepresst. Dadurch werden die Ionisierungseigenschaften weiter verbessert. Hierfür bietet sich ein Anpressgewicht im Bereich von 2 bis 3 kg, bevorzugt etwa 2,35 kg an.

Der elektrisch isolierende Behälter kann beispielsweise aus dielektrischen Material gebildet sein. Besonders geeignet ist ein aus Glas oder Keramik gebildeter Behälter. Insbesondere ist Glas geeignet, das eine Dielektrizitätszahl von 2 bis 16 und bevorzugt im Bereich von 6 bis 9 aufweist. So hat sich ein Glasmaterial mit einer Dielektrizitätszahl von 7,2 und einem dielektrischen Verlustfaktor von 70 × 10⁻⁴, gemessen bei einem Megahertz und 25 °C, als geeignet herausgestellt. Es bieten sich Flachbodengläser aus AR-Klarglas mit glatt verschmolzenem Rand an.

Vorteilhaft ist es, wenn der Behälter eine Wandstärke im Bereich von 0,5 bis 1,0 mm und bevorzugt im Bereich von 0,6 bis 0,8 mm hat. Geeignet ist beispielsweise ein Glasbehälter mit einer Länge von 30 mm ± 0,5, einem Außendurchmesser von 11,5 mm ± 0,14 und einer Wandstärke von 1,0 mm ± 0,03.

Die innere und/oder äußere Fläche des Behälters kann vor dem Einfüllen von Granulat mit einer Säure vorbehandelt werden. Damit wird vorteilhaft erreicht, dass die Oberfläche angeraut und offenporiger ist und das Glas eine größere Oberfläche und eine höhere Leitfähigkeit erhält.

Die Oberfläche des Behälters kann alternativ oder zusätzlich dazu auch beschichtet sein. Vorteilhaft ist in jedem Fall eine Entfettung der Oberflächen des Behälters, um Kriechströme zu verhindern.

Die Außenelektrode kann eine Gitterstruktur sein, die an dem Außenumfang des Behälters angebracht ist. Vorteilhaft ist hierzu eine Gittergröße im Bereich von Mesh 20 bis Mesh 38 und bevorzugt Mesh 30, d. h. die Siebgröße vorzugsweise im Bereich von 0,465 (Mesh 38) bis 0,85 mm (Mesh 20) und besonders bevorzugt im Bereich von 0,60 mm (Mesh 30) liegt.

Die Wahl der Gittergröße hat auch einen Einfluss auf den Ionisierungswirkungsgrad und sollte nicht zu klein, aber auch nicht zu groß sein. Geeignet ist beispielsweise eine Gitterstruktur (Mesh 30) aus einem Edelstahlmaterial Werkstoff Nr. 1,4301 mit einem Innendurchmesser von 11,3 mm, einer Maschenweite von 0,6 mm, einer Drahtstärke von 0,25 mm und einer Länge von 25 mm.

Die effektive Länge des Behälters liegt bevorzugt im Bereich von 25 bis 35 mm und beträgt vorzugsweise 30 mm.

Bei der optimalen Ausgestaltung der Vorrichtung kann der Ionisierungswirkungsgrad durch optimierte Proportionen verbessert werden. So hat sich ein Außendurchmesser von 11,5 mm ± 0,2 mm, eine Wandung von 0,7 mm ± 0,1 mm und eine Länge von 30 mm ± 0,5 für den Behälter als vorteilhaft herausgestellt. Die Außenelektrode umgibt dann im Bereich der Granulatfüllung den Behälter mit einer effektiven Länge von ca. 25 cm.

Die Ausgestaltung der Vorrichtung kann aber unabhängig hiervon vergrößert oder verkleinert werden, wobei die Beibehaltung der sogenannten Proportionen zwischen Außendurchmesser, Wandung und Länge beibehalten werden sollte.

Das Granulat kann mit einer Silikonscheibe abgedeckt werden. Hierzu bietet sich beispielsweise eine Dichtung aus dem Material UKTAsil 60T mit einem Außendurchmesser von 10,5 mm und einer Materialstärke von 1 mm und einem Loch in der Mitte für die Elektrodenleitung an.

Damit wird durch eine elektrische Isolierung eine Verunreinigung beispielsweise durch Feuchtigkeitseintrag oder Eintrag von weiteren Vergussmasse verhindert. Das abisolierte Ende einer die Innenelektrode bildenden Elektrodenleitung kann hierbei durch die Silikonscheibe durchgeführt werden, wobei der Silikonmantel der Elektrodenleitung an die Silikonscheibe angrenzt. So kann nach Einfüllen des Granulats die Elektrodenleitung der Innenelektrode mit dem abisolierten Ende und aufgesteckt in die Silikonschiebe in dem Behälter eingeführt werden, wobei da mithilfe des Silikonmantels der Elektrodenleitung und der daran angrenzenden Silikonschiebe das Granulat verpresst wird. Dies kann mit einer geeigneten Handhabungsvorrichtung mit einem vorgegebenen Anpressdruck erfolgen. Anschließend kann der Zwischenraum zwischen dem Behälter und der Innenwand des Behälters in dem von der Silikonscheibe nach außen mündenden Abschnitt des Behälters mit Vergussmaterial, wie beispielsweise einem Harzmaterial, ausgefüllt werden.

Das abisolierte Ende der mit einem Isolationsmaterial versehenen Elektrodenleitung kann verzinnt und zentrisch in den Innenraum des Behälters ausgerichtet sein. Dieses abisolierte Ende bildet dann die Innenelektrode der Vorrichtung.

Denkbar ist aber auch, dass das abisolierte Ende eines Litzenleiters so in das Granulat eingesteckt ist, dass die einzelnen Litzen unabhängig voneinander in dem Granulat aufgenommen und im Raum des Behälters verteilt sind. Somit können Litzen des abisolierten Endes der Elektrodenleitung voneinander räumlich beabstandet mit zwischenliegendem Granulatmaterial im Behälter angeordnet sein.

Geeignet sind beispielsweise mit Silikonisolierung versehene Hochspannungs-Lizenzleiter mit einem Leitungsquerschnitt von 1 mm² (AWG 15 bis 20, bevorzugt AWG 17, d.h. 19 Litzen á 0,25 mm²).

Die Erfindung wird nachfolgend anhand der beigefügten Zeichnungen anhand eines Ausführungsbeispiels näher erläutert. Es zeigen:
- Figur 1 -: Skizze einer Vorrichtung zur Ionisierung von Umgebungsluft;
- Figur 2 -: Skizze der Vorrichtung ohne Außenelektrode;
- Figur 3 -: Skizze einer Desinfektionseinrichtung mit Aerosolzusatz.

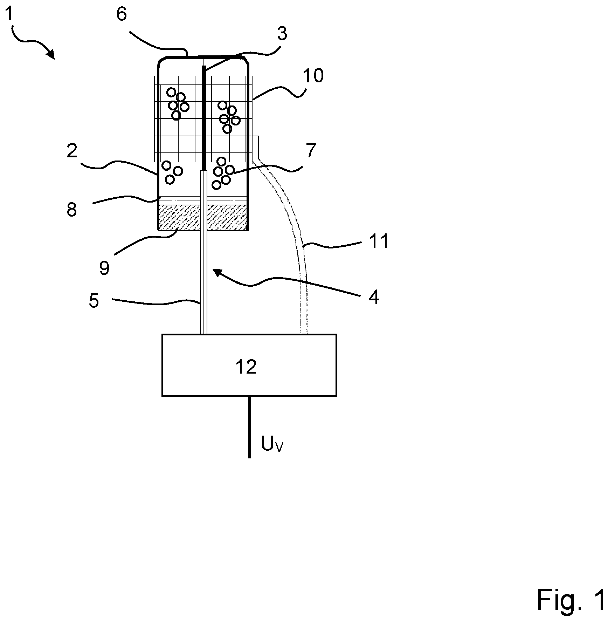

Figur 1 zeigt eine Skizze einer Vorrichtung 1 zur Ionisierung von Umgebungsluft. Die Vorrichtung 1 hat einen Behälter 2, der aus einem elektrisch isolierenden Material und bevorzugt aus einem dielektrischen Glas gebildet ist. In dem Innenraum des Behälters 2 ist eine Innenelektrode 3 angeordnet, die beispielsweise das abisolierte Ende einer Elektrodenleitung 4 ist, welche mit einem Isolationsmantel 5 umgeben ist. Als Isolationsmantel 5 ist Silikonmaterial geeignet, so kann eine Elektrodenleitung mit einer Silikonisolierung genutzt werden, die im Bereich von mindestens 10 kV und bevorzugt eine Hochspannungsfestigkeit von 20 kV und mehr aufweist.

Der Behälter 2 ist am oberen Ende durch einen Boden 6 verschlossen und am gegenüberliegenden Ende offen. Das absiolierte Ende 3 der Elektrodenleitung 4 wird von der offenen Seite des Behälters 2 in Richtung Boden 6 eingeführt und ist bevorzugt mit üblichen Toleranzen zentriert angeordnet.

In den Innenraum des Behälters ist zwischen der Innenwand und der Innenelektrode 3 ein Granulat 7 angeordnet. Zum freien Ende des Behälters 2 hin ist die Füllung aus Granulat 7 dann mit einer Silikonscheibe 8 abgedeckt. Es ist erkennbar, dass das abisolierte Ende 3 der Elektrodenleitung ebenfalls mit einem Teil einer Isolierung durch die Silikonscheibe 8 hindurch geführt wird. Zur Vermeidung von Kriechströmen und Verhinderung von Überschlag ist es vorteilhaft, wenn ein Teil des gegebenenfalls im Durchmesser verjüngten Isolationsmantels 5 auch durch die Silikonscheibe 8 hindurch geführt wird und sich von dort noch wenige Millimeter in das Granulat 7 hinein erstreckt.

Der weitere Zwischenraum zwischen der Ausmündung der Innenwand des Behälters 2 und dem Isolationsmantel 5 der Elektrodenleitung 4 kann dann mit einem elektrisch isolierendem Füllmaterial 9, wie beispielsweise einem Harz, ausgefüllt werden. Damit kann die Elektrodenleitung zusammen mit der Silikonscheibe 8 und dem Granulat 7 fest in dem Behälter 2 gehalten werden. Mit dem Füllmaterial 9 gelingt es zusammen mit der Silikonscheibe 8, das Eindringen von Verunreinigung, beispielsweise Feuchtigkeit, in das Granulat 7 sicher zu verhindern.

Am Außenumfang des Behälters 2 ist eine Außenelektrode 10 angeordnet. Diese kann auch aus einem Gittermaterial sein, d. h. den Behälter 2 konzentrisch umgibt und von Ausmündung zum Ende des Behälters 2 zum Boden 6 hin gesehen eine Länge hat, die sich über einen wesentlichen Teil der Länge der Granulatfüllung 7 erstreckt. Die Länge der Außenelektrode sollte im Bereich von zwei Dritteln der Länge der Granulatfüllung, d. h. der effektiven Länge der Vorrichtung 1, betragen.

Erkennbar ist, dass die Außenelektrode 10 in dem dargestellten Ausführungsbeispiel als Gitterstruktur ausgebildet ist. Die Gittergröße sollte im Bereich von Mesh 20 bis Mesh 38, bevorzugt Mesh 30 betragen. Dies entspricht einer Siebgröße im Bereich von 0,85 bis 0,456 mm, bevorzugt 0,60 mm.

An die Außenelektrode 10 ist eine Außenelektrodenleitung 11 angeschlossen, die zusammen mit der Innenelektrodenleitung 4 mit einer Ansteuerelektronik 12 verbunden ist. Diese Ansteuerelektronik 12 mit einer Versorgungsspannung Uv versorgt und ist eingerichtet, die Innen- und Außenelektrode 3, 10 mit einer Hochspannung zu beaufschlagen.

Die an die Innen- und Außenelektrode 3, 10 angelegte Hochspannung sollte weniger als 2 kV betragen und bevorzugt im Bereich von 1.500 bis 1.800 V, bzw. bevorzugt etwa 1.750 V betragen. Damit wird die Entstehung von schädlichen Stickoxiden NOx verhindert und die Umgebungsluft so ionisiert, dass Hydroxylradikale gebildet werden.

Bei einer höheren Spannung im Bereich von etwa 2 bis 3 kV erfolgt die Ionisierung von Umgebungsluft durch Bildung von Ozon. Eine höhere Spannung von mehr als 3 kV führt dann zu schädlicher Bildung von Stickoxiden NOx.

Die mit der Vorrichtung ionisierte Umgebungsluft kann dann auf vielfältige Weise zu Entkeimung genutzt werden.

So kann die Vorrichtung eingesetzt werden, um Raumluft von schädlichen Bakterien, Viren und Pilzsporen zu reinigen. Besonders vorteilhaft ist es aber, wenn die Vorrichtung eingesetzt wird, um einen ionisierten Luftstrom zu erzeugen, in den dann Aerosole eingebracht werden, und wenn dieser aerosolhaltige, ionisierte Luftstrom dann zu Entkeimung weiter verwendet wird. Die Vorrichtung kann in diesem Zusammenhang zu Patientenversorgung genutzt werden, um beispielsweise mit einem Beatmungsschlauch aerosolhaltige ionisierte Luft in die Lunge und insbesondere in die Alveolargänge der Lunge zu transportieren, um auf diese Weise bakterielle und virale Erkrankungen der Lunge zu behandeln.

Hierzu sollte die Luft angeheizt werden, aber nicht mehr als 37 °C betragen. Die Partikelgröße der Aerosole soll möglichst klein sein und im Bereich von 0,2 bis 0,4 µm betragen. Auf diese Weise wird sichergestellt, dass der Wassereintrag nicht übermäßig groß ist.

Mithilfe der Aerosole wird im Zusammenhang mit der ionisierten Luft ein schädliches Austrocknen der Haut verhindert.

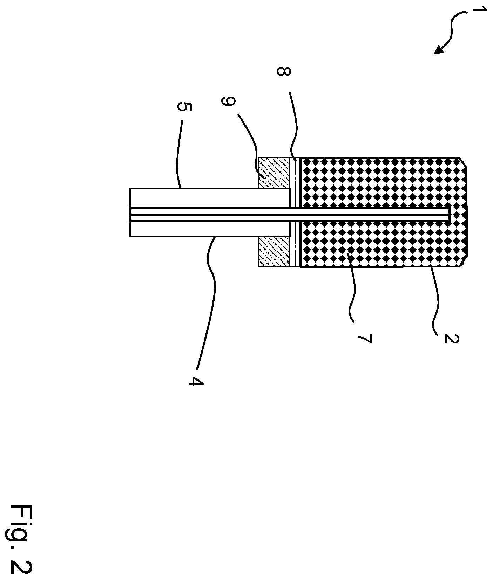

Figur 2 zeigt eine Skizze eines Teils der Vorrichtung 1 mit dem in den Behälter 2 eingeführten Granulat 7.

Deutlich wird, dass die Innenelektrode 3 das abisolierte Ende eines elektrischen Leiters 4 bildet, der durch einen Silikonmantel 5 umgeben ist. Dieser Silikonmantel 5 liegt mit seiner Stirnseite auf der Silikonscheibe 8 auf, sodass nach Einfüllen des Granulates 7 in den Behälter 2 das Granulat 7 durch die Silikonscheibe 8 mittels Krafteinwirkung über den Silikonmantel 5 zusammengepresst wird. Der dennoch verbleibende Zwischenraum von dem Silikonmantel 5 bis zum freien Ende des Behälters 2 wird dann mit Vergussmaterial 9 ausgefüllt.

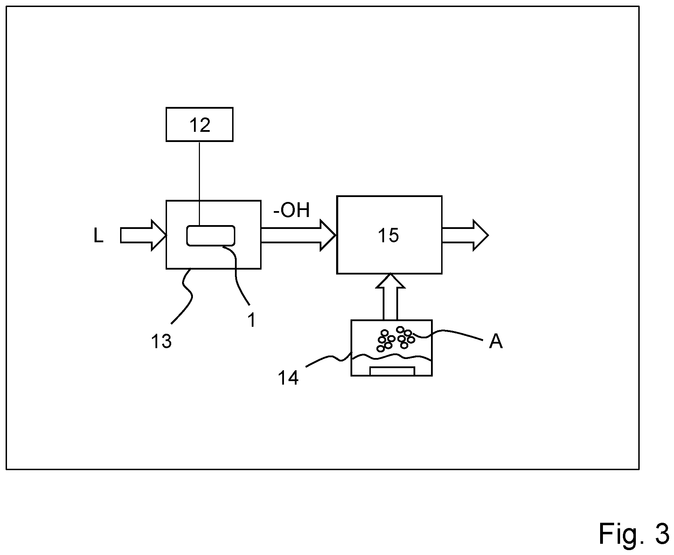

Figur 3 zeigt eine Skizze einer Desinfektionsvorrichtung mit der vorbeschriebenen Ionisierungsvorrichtung 1 und einer Mischbox 15 zur Aerosolzuführung.

In einem Rohrstück 13 oder Behälter ist die Ionisierungsvorrichtung 1 angeordnet. Die Umgebungsluft L wird bspw. mit einem nicht gezeigten Lüfter durch das Rohrstück 13 geleitet und durch Betrieb der Ionisierungsvorrichtung 1 so ionisiert, dass Hydroxylradikale -OH entstehen. Diese werden in eine Mischbox 15 geführt, in die weiterhin feine wasserhaltige Aerosole strömen.

Es ist vorteilhaft, insbesondere für medizinische Anwendungen mit Zufuhr in die Atemwege, dass die Aerosole eine Partikelgröße von weniger als 0,4 µm haben.

Die Aerosole werden in einem Aerosolgenerator 14 aus dort eingeführtem Wasser oder wässriger Lösung, insb. reines Wasser, erzeugt. Dies kann durch Ultraschallvernebelung, Zerstäubung mittels Druckluft und dergleichen erfolgen.

Das mit Hydroxylradikalen angereicherte, aerosolhaltige Luftgemisch wird dann zur Desinfektion ausgeleitet. Es kann in ein Gehäusevolumen bspw. zur Handdesinfektion geleitet werden, in das Raumvolumen eines Gebäuderaumes zur Luftreinigung, in einen Tubus zum Einleiten in Atemwege eines Patienten etc.

## Patentansprüche

1. Vorrichtung (1) zur Ionisierung von Umgebungsluft mit einem Behälter (2) aus elektrisch isolierendem Material, einer im Innenraum des Behälters (2) angeordneten Innenelektrode (3) und einer an der Außenseite des Behälters (2) angeordneten Außenelektrode (10) , mit einer Ansteuerelektronik (12), die mit der Innenelektrode (3) und der Außenelektrode (10) zur Beaufschlagung mit einer Versorgungsspannung verbunden ist,
**gekennzeichnet durch**
ein elektrisch leitfähiges Granulat (7) mit einer mittleren Korngröße im Bereich von 10 bis 450 µm in dem Innenraum des Behälters (2), wobei das Granulat (7) bei eingesteckter Innenelektrode (3) zusammengepresst und die Innenelektrode (3) von dem Granulat (7) umgeben ist.

2. Vorrichtung (1) nach Anspruch 1, **dadurch gekennzeichnet, dass** das Granulat (7) aluminiumhaltige, kupferhaltige, edelstahlhaltige, titanhaltige, graphithaltige und/oder magnesiumhaltige Partikel enthält.

3. Vorrichtung (1) nach Anspruch 1 oder 2, **dadurch gekennzeichnet, dass** das Granulat (7) ein Kupferpulver mit einer Korngrößenverteilung im Bereich von 10 bis 450 µm ist.

4. Vorrichtung (1) nach Anspruch 1 oder 2, **dadurch gekennzeichnet, dass** das Granulat (7) ein Aluminiumpulver oder Edelstahlpulver mit einer Kerngröße von 10 bis 450 µm ist.

5. Vorrichtung (1) nach einem der vorhergehenden Ansprüche, **dadurch gekennzeichnet, dass** der Behälter (2) aus einem dielektrischen Material, bevorzugt aus Glas oder Keramik gebildet ist.

6. Vorrichtung (1) nach einem der vorhergehenden Ansprüche, **dadurch gekennzeichnet, dass** der Behälter (2) eine Wandstärke im Bereich von 0,5 bis 1,0 mm, bevorzugt im Bereich von 0,6 bis 0,8 mm hat.

7. Vorrichtung (1) nach einem der vorhergehenden Ansprüche, **dadurch gekennzeichnet, dass** die innere und/oder äußere Oberfläche des Behälters (2) säurebehandelt ist.

8. Vorrichtung (1) nach einem der vorhergehenden Ansprüche, **dadurch gekennzeichnet, dass** die effektive Länge des Behälters (2) im Bereich von 25 bis 35 mm liegt und bevorzugt 30 mm beträgt.

9. Vorrichtung (1) nach einem der vorhergehenden Ansprüche, **dadurch gekennzeichnet, dass** die Länge der Außenelektrode (10) im Bereich von 20 bis 30 mm liegt und bevorzugt 25 mm beträgt.

10. Vorrichtung (1) nach einem der vorhergehenden Ansprüche, **dadurch gekennzeichnet, dass** das Granulat (7) mit einer Silikonscheibe (8) abgedeckt ist und eine Elektrodenleitung (4) durch die Silikonscheibe (8) geführt ist, wobei ein Silikonmantel (5) der Elektrodenleitung (4) an die Silikonscheibe (8) angrenzt.

## Claims

1. Device (1) for ionising ambient air with a container (2) made of electrically insulating material, an inner electrode (3) arranged in the interior of the container (2) and an outer electrode (10) arranged on the outside of the container (2), with control electronics (12) which are connected to the inner electrode (3) and the outer electrode (10) for the application of a supply voltage,
**characterised by**
an electrically conductive granulate (7) with an average grain size in the range from 10 to 450 µm in the interior of the container (2), wherein the granulate (7) is compressed when the inner electrode (3) is inserted and the inner electrode (3) is surrounded by the granulate (7).

2. Device (1) according to claim 1, **characterised in that** the granulate (7) contains aluminium-containing, copper-containing, stainless steel-containing, titanium-containing, graphite-containing and/or magnesium-containing particles.

3. Device (1) according to claim 1 or 2, **characterised in that** the granulate (7) is a copper powder with a particle size distribution in the range from 10 to 450 µm.

4. Device (1) according to claim 1 or 2, **characterised in that** the granulate (7) is an aluminium powder or stainless-steel powder with a core size of 10 to 450 µm.

5. Device (1) according to one of the preceding claims, **characterised in that** the container (2) is formed from a dielectric material, preferably glass or ceramic.

6. Device (1) according to one of the preceding claims, **characterised in that** the container (2) has a wall thickness in the range from 0.5 to 1.0 mm, preferably in the range from 0.6 to 0.8 mm.

7. Device (1) according to one of the preceding claims, **characterised in that** the inner and/or outer surface of the container (2) is acid-treated.

8. Device (1) according to one of the preceding claims, **characterised in that** the effective length of the container (2) is in the range from 25 to 35 mm and is preferably 30 mm.

9. Device (1) according to one of the preceding claims, **characterised in that** the length of the outer electrode (10) is in the range from 20 to 30 mm and is preferably 25 mm.

10. Device (1) according to one of the preceding claims, **characterised in that** the granulate (7) is covered with a silicone disc (8) and an electrode line (4) is guided through the silicone disc (8), a silicone sheath (5) of the electrode line (4) adjoining the silicone disc (8).

## Revendications

1. Dispositif (1) d'ionisation de l'air ambiant, comprenant un récipient (2) en matériau électriquement isolant, une électrode intérieure (3) disposée dans l'espace intérieur du récipient (2), une électrode extérieure (10) disposée sur le côté extérieur du récipient (2), et une électronique de commande (12) qui est reliée à l'électrode intérieure (3) et à l'électrode extérieure (10) pour appliquer une tension d'alimentation,
**caractérisé par**
des granulés électriquement conducteurs (7), ayant une granulométrie moyenne dans la plage de 10 à 450 µm, dans l'espace intérieur du récipient (2), les granulés (7) étant comprimés, lorsque l'électrode intérieure (3) est introduite, et l'électrode intérieure (3) étant entourée par les granulés (7).

2. Dispositif (1) selon la revendication 1,
**caractérisé en ce que** les granulés (7) comprennent des particules contenant de l'aluminium, du cuivre, de l'acier inoxydable, du titane, du graphite et/ou du magnésium.

3. Dispositif (1) selon la revendication 1 ou 2,
**caractérisé en ce que** les granulés (7) sont du cuivre en poudre ayant une distribution granulométrique dans la plage de 10 à 450 µm.

4. Dispositif (1) selon la revendication 1 ou 2,
**caractérisé en ce que** les granulés (7) sont de l'aluminium en poudre ou de l'acier inoxydable en poudre ayant une taille de noyau comprise entre 10 et 450 µm.

5. Dispositif (1) selon l'une des revendications précédentes,
**caractérisé en ce que** le récipient (2) est réalisé en un matériau diélectrique, de préférence en verre ou en céramique.

6. Dispositif (1) selon l'une des revendications précédentes,
**caractérisé en ce que** le récipient (2) a une épaisseur de paroi dans la plage de 0,5 à 1,0 mm, de préférence dans la plage de 0,6 à 0,8 mm.

7. Dispositif (1) selon l'une des revendications précédentes,
**caractérisé en ce que** la surface intérieure et/ou extérieure du récipient (2) est traitée à l'acide.

8. Dispositif (1) selon l'une des revendications précédentes,
**caractérisé en ce que** la longueur effective du récipient (2) est dans la plage de 25 à 35 mm et est de préférence de 30 mm.

9. Dispositif (1) selon l'une des revendications précédentes,
**caractérisé en ce que** la longueur de l'électrode extérieure (10) est dans la plage de 20 à 30 mm et est de préférence de 25 mm.

10. Dispositif (1) selon l'une des revendications précédentes,
**caractérisé en ce que** les granulés (7) sont recouverts d'un disque de silicone (8), et une ligne d'électrode (4) traverse le disque de silicone (8), une gaine de silicone (5) de la ligne d'électrode (4) étant adjacente au disque de silicone (8).
